# EUROPEAN PATENT APPLICATION

(11) **EP 4 844 329 A1**
(43) Date of publication of application: **25.11.2026**
(21) Application number: 25305723.6
(22) Date of filing: 20.05.2025
(51) Int. Cl.: G01B 5/28

(54) **TOOL FOR MEASURING UNDER-FLANGE FLATNESS ON A MEDICAL INJECTION DEVICE BARREL**

(71) Applicant: Becton Dickinson France, 38800 Le Pont-de-Claix (FR)
(72) Inventor: HELL, Julien, 38800 LE PONT-DE-CLAIX (FR); SILLARD, Yann, 38800 LE PONT-DE-CLAIX (FR)
(74) Representative: Germain Maureau

(57) **Abstract**

Provided herein is a tool for measuring under-flange flatness on a barrel of a medical injection device. The tool includes a base, a barrel holder, a contact sensor comprising a sensor element positioned adjacent the flange of the barrel when the barrel is retained by the barrel holder, with the contact sensor configured to measure a distance between the sensor element and an underside of flange, so as to obtain a measurement value, and a digital comparator operatively connected to the contact sensor to receive measurement values therefrom. The contact sensor obtains a plurality of measurement values as the barrel is rotated on the barrel holder, with the digital comparator receiving the measurement values from the sensor, determining maximal - minimal measurement values from the plurality of measurement values, and calculating an under-flange flatness measurement for the flange from the maximal - minimal measurement values.

## Description

### BACKGROUND OF THE INVENTION

### Field of the Invention

The present disclosure relates generally to medical injection devices and, more particularly, to a tool used for measuring the underside flatness of the flange on a barrel of a medical injection device.

### Description of Related Art

Medical injection devices, such as syringes or other injection devices that include a cartridge or vial therein, are used in a variety of environments for administering liquids (e.g., medications or drugs) to a patient. As a primary example, syringes may be provided as prefilled syringes, which provide the convenience of rapidly delivering the liquid therein to a patient without the need to first aspirate the medication from another container and meter its volume. For dispensing fluids, the prefilled syringe will typically include a barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity of a syringe when the stopper is inserted into the syringe.

Components of the syringes (or other injection devices) as described above often need to be transported during or after manufacturing. As one example, barrels of the injection devices may be transported from a manufacturing site to a site at which the barrels may be filled with a medication, with stoppers then inserted into the barrels to seal the medication within the barrel, with such filling and stoppering of the barrels being performed by an automated fill and finish machine. As another example, and upon completion of filling and stoppering of the barrel, the containers may be visually inspected by a high-speed automated system to detect defects that could be detrimental to the final product performance.

When barrels are handled and conveyed as part of any of the processes described above, it is recognized that the configuration/construction of the barrel is important to achieving a high throughput of an automated handling system or conveyor. As one example, the flatness of an underside of the flange on the barrel must be within a desired tolerance range in order that the barrel fits properly within a holding device of the automated handling system or conveyor, both in order to achieve high-speed operation of the automated handling system or conveyor and to avoid rejects. With such considerations in mind, it is desirable to ensure that the under-flange flatness is within a desired tolerance range.

Accordingly, a need exists in the art for a means for quickly and accurately measuring under-flange flatness on a barrel.

### SUMMARY OF THE INVENTION

Provided herein is a tool for measuring under-flange flatness of a medical injection device barrel having a cylindrical main body and a flange positioned at a proximal end of the cylindrical main body. The tool includes a base and a barrel holder positioned on the base and configured to retain the barrel therein, the barrel holder preventing movement of the barrel in a distal-proximal direction and enabling rotation of the barrel about a longitudinal axis of the barrel. The tool also includes a contact sensor positioned on the base, the contact sensor comprising a sensor element positioned adjacent the flange of the barrel when the barrel is retained by the barrel holder, with the contact sensor configured to measure a distance between the sensor element and an underside of flange, so as to obtain a measurement value. The tool further includes a digital comparator operatively connected to the contact sensor to receive measurement values therefrom. The contact sensor obtains a plurality of measurement values as the barrel is rotated on the barrel holder. The digital comparator receives the plurality of measurement values from the sensor, determines maximal - minimal measurement values from the plurality of measurement values, and calculates an under-flange flatness measurement for the flange from the maximal - minimal measurement values.

In certain configurations, the base comprises a base plate and an end plate positioned at one end of the base plate and extending upwardly therefrom.

In certain configurations, the barrel holder comprises a plurality of bearings provided on the base plate and arranged to engage the cylindrical main body, a wheel suspended above the base plate and positioned above the plurality of bearings such that the barrel is positionable between the plurality of bearings and the wheel, with the wheel configured to engage the cylindrical main body to enable rotation thereof on the plurality of bearings responsive to rotation of the wheel, and a stop flange provided at one end of the plurality of bearings, opposite from the end plate, the stop flange configured to engage a top side of the flange when the barrel is positioned on the plurality of bearings. When loaded on the barrel holder, the barrel is engaged by the stop flange and the end plate at opposing ends thereof, to prevent movement of the barrel in the distal-proximal direction.

In certain configurations, the wheel is mounted on a support arm, with the support arm supported and retained by an arm holder affixed to the base plate, and wherein the wheel is rotatably mounted on the support arm via a wheel bearing.

In certain configurations, the wheel comprises an outer rubber surface configured to grip the cylindrical main body, to facilitate rotation of the barrel when the wheel is rotated.

In certain configurations, the stop flange is positioned at a distal end of a spring-loaded arm configured to move the stop flange in the distal-proximal direction to selectively bring the stop flange into and out of contact with the flange of the barrel.

In certain configurations, the spring-loaded arm is mounted retained on a mounting block that is secured on the base plate, with the spring-loaded arm extending through an opening of the mounting block and movable relative to the mounting block in the distal-proximal direction.

In certain configurations, the spring-loaded arm comprises a handle positioned at a proximal end of a spring-loaded arm, the handle configured to be engaged by a user, and a spring is positioned about the spring-loaded arm at a location between the mounting block and the stop flange, with the spring biasing the arm to a distal location where the stop flange is brought into contact with the flange of the barrel.

In certain configurations, the plurality of bearings comprises a distal pair of bearings and a proximal pair of bearings.

In certain configurations, the contact sensor comprises a sensor key on which the sensor element is mounted, with the sensor key mounted on rods secured to the end plate.

In certain configurations, the sensor key is repositionable along a length of the rods in order to properly position the sensor element relative to the underside of the flange.

In certain configurations, the digital comparator is configured generate an output indicating that the barrel is defective if the under-flange flatness measurement falls outside of an acceptable tolerance range.

In certain configurations, the acceptable tolerance range of the under-flange flatness measurement is +/- 0.4 mm.

Also provided herein is a method for measuring under-flange flatness of a barrel using a tool. The method includes loading the barrel into the barrel holder of the tool, activating the contact sensor to ready the contact sensor for acquisition of measurements on the under-flange flatness of the barrel, rotating the barrel in the barrel holder a full 360° about a longitudinal axis of the barrel and, as the barrel is being rotated about the longitudinal axis, continuously acquiring measurement values of the under-flange flatness of the barrel via the contact sensor. The method also includes providing the measurement values to the digital comparator, with the digital comparator analyzing the measurement values via the digital comparator to determine maximal - minimal values and generating an under-flange flatness measurement for the flange from the maximal - minimal values.

In certain configurations, loading the barrel into the barrel holder comprises actuating the support arm to change the positioning of the wheel and provide access to the plurality of bearings, actuating the spring-loaded arm by pulling the handle to move the stop flange proximally and provide access to the plurality of bearings, positioning the barrel onto the plurality of bearings and with a tip of the barrel in contact with an end plate of the base and, upon such placement of the barrel onto the plurality of bearings, releasing the spring-loaded arm to bring the stop flange into contact with the flange of the barrel and actuating the support arm to bring the wheel into contact with the cylindrical main body of the barrel.

### BRIEF DESCRIPTION OF THE DRAWINGS

FIG. 1 is a perspective view of a barrel with which embodiments of the disclosure may be implemented;
FIG. 2 is a perspective view of a tool for measuring under-flange flatness of a barrel, according to a non-limiting embodiment described herein;
FIG. 3 is a top view of the tool of FIG. 2; and
FIG. 4 is a side cross-sectional view of the tool of FIG. 2.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding an injection device in preparation for or during use. Similarly, in this application, the terms "in the distal direction" and "distally" mean in the direction toward the distal tip of the injection device, and the terms "in the proximal direction" and "proximally" mean in the direction opposite the direction of the distal tip of the injection device.

Aspects and embodiments of the disclosure are directed to a tool for measuring under-flange flatness on a barrel, for purposes of quality control when manufacturing the barrel and ensuring that the under-flange flatness is within a desired tolerance range. A barrel may be inserted into the tool, to obtain a measurement of the under-flange flatness of the barrel.

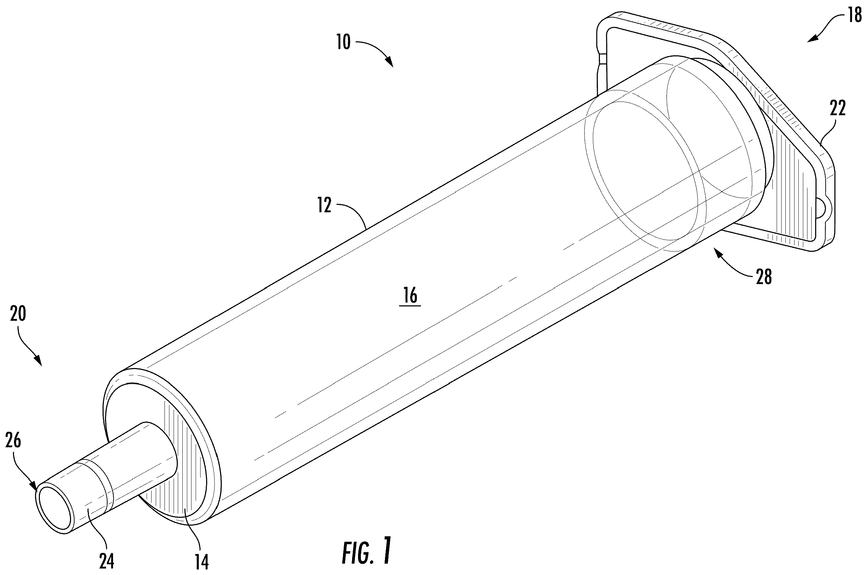

Referring to FIG. 1, shown is a non-limiting embodiment of a medical injection device barrel 10 with which aspects or embodiments of the disclosure may be implemented. The barrel 10 may comprise a syringe barrel, cartridge, or vial that may be part of a syringe or other injection device, according to embodiments of the disclosure. When barrel 10 is provided as a syringe barrel, it is recognized that the overall syringe would also include a plunger assembly (not shown), with the plunger assembly being axially movable within the barrel 10 to an advanced position to facilitate administering of an injectable fluid (e.g., medication) to a patient, for example. As known in the art, the plunger assembly may include a plunger rod having a stopper attached to a distal end thereof, with stopper formed from a material that is different from the material of the plunger rod, such as rubber or another elastomeric material, and that is capable of forming a tight seal with the barrel 10 as it is advanced therethrough.

The barrel 10 is formed of a cylindrical main body 12 and an end wall 14 that collectively define a chamber 16 for retaining fluid therein. The barrel 10 includes an open proximal end 18 configured to receive a plunger assembly therein and a distal end 20 at which end wall 14 is positioned. The proximal end 18 of the barrel 10 may include a flange 22 to facilitate handling and positioning of the barrel 10 and to maintain the relative position of the barrel 10 with respect to the plunger assembly during medication administration. In one embodiment, the distal end 20 of barrel 10 may include a tip 24 that extends distally outward from the end wall 14 and defines an opening or lumen 26 in fluid communication with the chamber 16. In some embodiments, and as illustrated in FIG. 1, the tip 24 is configured as a luer connection or luer fitting that may mate with a corresponding luer connection or luer fitting of a vascular access device (when administering fluid from the barrel) or a fluid source (when aspirating fluid into the barrel). It can be appreciated that, in other embodiments, tip 24 may be provided as a hub within which a needle is secured (i.e., a staked needle syringe) and extends out distally therefrom.

As previously described, it is known in the art that barrels must be manufactured to high quality standards, so that critical dimensions of the barrel - such as the under-flange flatness of the barrel - are within a desired tolerance range. The controlling of the under-flange flatness of the barrel ensures that the barrel fits properly into an associated holding device of an automated handling system or conveyor during processing (e.g., during a fill and finish processing operation or a visual inspection), such as an endless screw or star wheel, as non-limiting examples.

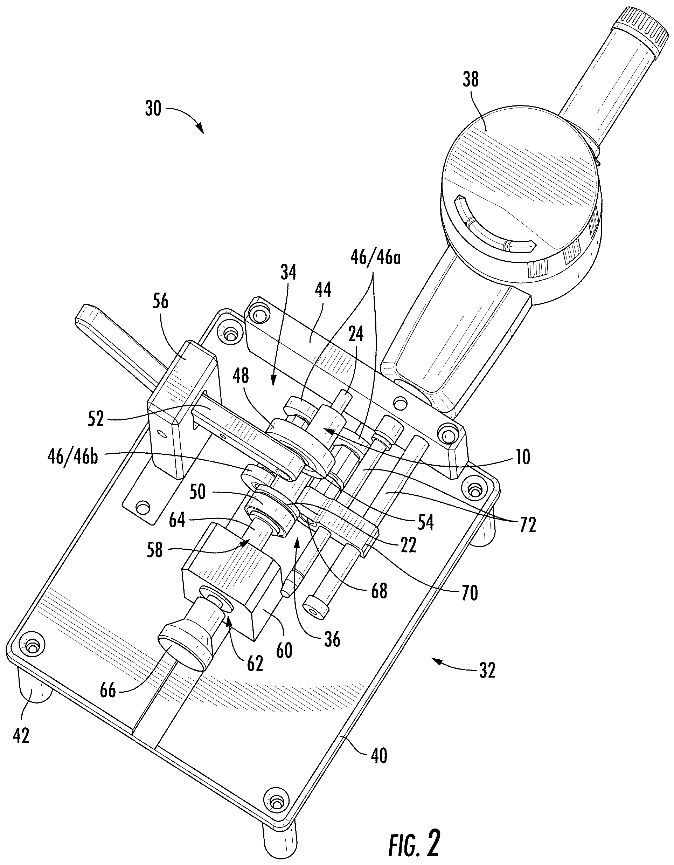

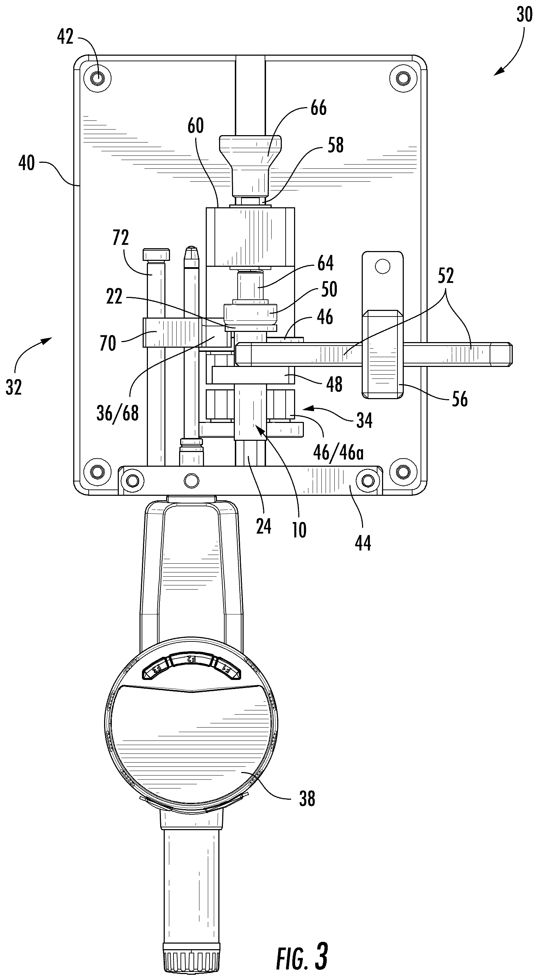

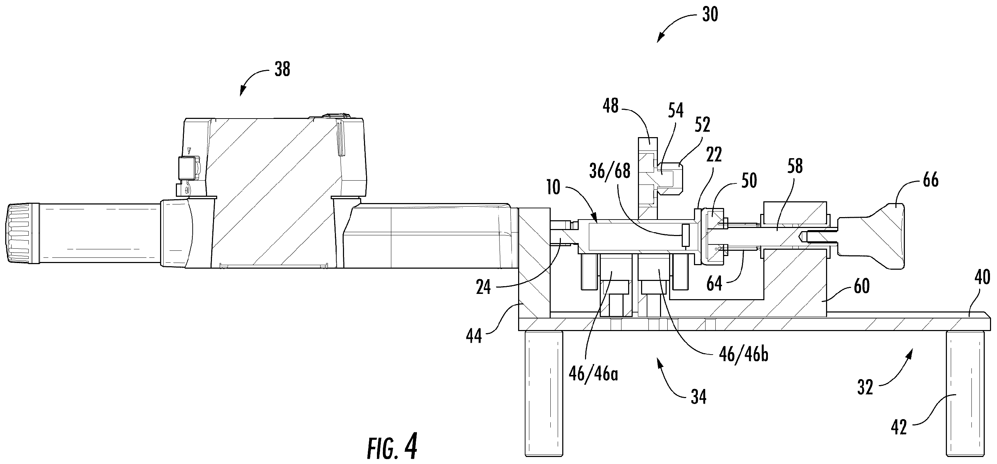

Referring now to FIGS. 2-4, a barrel inspecting tool 30 (hereafter "tool 30") is illustrated according to an aspect of the disclosure. The tool 30 generally comprises a base 32, a barrel holder 34, a sensor 36, and a digital comparator 38.

The base 32 includes a base plate 40 supported on a plurality of legs 42, along with an end plate or "tip stop" 44 that extends upwardly from the base plate 40. The base 32 provides a stable platform or frame for the tool 30 and also supports and arranges the barrel holder 34, sensor 36, and digital comparator 38, in order to facilitate measuring of the barrel 10.

The barrel holder 34 comprises a plurality of components that collectively retain the barrel 10 in place and provide for rotation thereof when acquiring measurements of the under-flange flatness of the barrel 10. The barrel holder 34 comprises a plurality of bearings 46, a wheel 48, and a stop flange 50, along with associated components, as explained in further detail below.

The bearings 46 are provided on base plate 40 and arranged so as to support the cylindrical main body 12 of barrel 10, with the cylindrical main body 12 resting on the bearings 46 and being rotatable thereon. In the illustrated embodiment, an arrangement of four (4) bearings 46 is provided on tool, including a pair of distal bearings 46a and a pair of proximal bearings 46b.

The wheel 48 is suspended above the base plate 40, so as to be positioned above the barrel 10 at a location adjacent the bearings 46. The wheel 48 may be positioned at a location in the longitudinal direction (i.e., proximally and distally) that is between the distal pair of bearings 46a and the proximal pair of bearings 46b. The wheel 48 is rotatably mounted on a support arm 52 (via a wheel bearing 54), with the support arm 52 in-turn being supported and retained by an arm holder 56. The arm 52 may be actuated by a user to change the positioning of wheel 48, i.e., bring the wheel 48 into and out of contact with barrel 10, in order to provide for mounting a barrel 10 onto tool 30 for testing and removal of a barrel 10 from tool 30 after testing. In use of tool 30, the wheel 48 may be brought into contact with the cylindrical main body 12 of barrel 10 in order maintain contact between the barrel 10 and the bearings 46, to provide for a smooth rotation of the barrel 10 during testing/measurement. When in contact with the barrel 10, the wheel 48 is rotated (either manually rotated, in a preferred embodiment, or via a servo motor, in another embodiment) by a user to cause a corresponding rotation of the barrel 10, to enable the acquisition of measurements of the under-flange flatness. In some embodiments, wheel 48 includes an outer rubber surface or padding that allows for the wheel 48 to better grip the cylindrical main body 12 and rotate the barrel 10 when turned, while also preventing damage to the barrel 10.

The stop flange 50 is provided at one end of bearings 46, adjacent to a location of where the flange 22 of barrel 10 is positioned when loaded into tool 30. The stop flange 50 is provided on a spring-loaded arm 58 that is movable in a longitudinal direction (i.e., proximally and distally). The arm 58 is retained on a mounting block 60 that is secured on base plate 40, with the arm 58 extending through an opening 62 of mounting block 60. A spring 64 is positioned about the arm 58 at a location between the mounting block 60 and stop flange 50, with the spring 64 biasing the arm 58 to a location where the stop flange 50 is in contact with the flange 22 of barrel 10. In use of tool 30, a user may pull on a handle 66 of spring-loaded arm 58 to draw the arm 58 back proximally (overcoming a bias of spring 64), allowing for a barrel 10 to be mounted on tool 30. Once the barrel 10 is mounted on tool 30, the user may then release the handle 66, with the spring 64 biasing the stop flange 50 into contact with the flange 22 of barrel 10, which functions to maintain the barrel 10 in an appropriate position (i.e., with tip 24 of barrel 10 in contact with end plate 44) for testing/measurement of the under-flange flatness.

The sensor 36 of tool 30 is configured as a contact sensor that operates to determine the under-flange flatness of the barrel flange 22. The sensor 36 may comprise a sensor element 68 mounted on a sensor key 70, with the sensor key 70 in turn mounted on rods 72 secured to base 32 (i.e., rods 72 are mounted on end plate 44). According to embodiments, the sensor key 70 may be repositioned along a length of rods 72 in order to properly position the sensor element 68 relative to the underside of flange 22, with repositioning of the sensor key 70 on rods 72 thus enabling use of the tool 30 with multiple barrel sizes. The sensor 36 operates to acquire continuous values (of a distance between sensor element 68 and the underside of flange 22) during rotation of the barrel 10 (via wheel 48), with these values provided to digital comparator 38, as described in further detail below.

The digital comparator 38 is operably connected to sensor 36 to receive measurement values therefrom. In real time, as the barrel 10 is rotated 360° on tool 30 via actuation of wheel 48, the digital comparator 38 receives measured values from the sensor 36, from which maximal - minimal values are determined/calculated by the digital comparator 38. Based on the calculated maximal - minimal values, the digital comparator 38 generates an under-flange flatness measurement for the flange 22. According to embodiments, it is desired for the measured under-flange flatness to vary by no more than +/- 0.4 mm (and preferably less), in order for the barrel 10 to be considered to fall within acceptable manufacturing tolerances.

In some embodiments, if the under-flange flatness measurement calculated by the digital comparator 38 falls outside of an acceptable tolerance range, the digital comparator 38 may generate an output indicating that the barrel 10 is defective. As previously described, a barrel 10 with an unacceptable under-flange flatness may not fit properly within a holding device of an automated handling system or conveyor, which can prevent high-speed operation of the automated handling system or conveyor, such as by jamming up to system/conveyor.

In use of the tool 30, a barrel 10 may first be loaded into tool 30. To enable loading of the barrel 10 into tool 30, support arm 52 including wheel 48 thereon may be actuated by a user to change the positioning of wheel 48 and provide access to the bearings 46, while spring-loaded arm 58 may be actuated proximally by the user (by pulling handle 66 proximally) to also provide access to the bearings 46.

With the support arm 52 and spring-loaded arm 58 positioned as described above, the barrel 10 may be positioned on bearings 46 and with tip in contact with end plate 44. Upon such placement of the barrel 10, the spring-loaded arm 58 may be released by the user to bring the stop flange 50 into contact with the flange 22 of barrel 10 (i.e., with top surface of flange 22), while the support arm 52 may also be actuated to bring the wheel 48 into contact with the cylindrical main body 12 of barrel 10.

With the barrel 10 loaded onto tool 30 as described above, the contact sensor 36 may be armed/activated and begin acquiring measurements of the under-flange flatness of barrel 10. After arming/activating of the sensor 36, the barrel 10 is rotated about a longitudinal axis of the barrel 10, with such rotation performed by actuation of the wheel 48, which in turn causes rotation of the barrel 10. The wheel 48 may be actuated manually by the user to cause a full 360° rotation of the barrel 10. As the barrel 10 is rotated a full 360°, the sensor 36 operates to continuously acquire measurement values (of a distance between sensor element 68 and the underside of flange 22). These measurement values are provided to the digital comparator 38, with the digital comparator 38 determining/calculating maximal - minimal values from which the digital comparator 38 generates an under-flange flatness measurement for the flange 22.

Beneficially, embodiments of the invention thus provide a tool for quickly and accurately measuring under-flange flatness on a barrel. The tool provides for securing of the barrel in the tool and for a 360° rotation of the barrel, so as to enable measuring of the under-flange flatness by a contact sensor and associated digital comparator provided on the tool.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A tool for measuring under-flange flatness of a medical injection device barrel having a cylindrical main body and a flange positioned at a proximal end of the cylindrical main body, the tool comprising:
a base;
a barrel holder positioned on the base and configured to retain the barrel therein, the barrel holder preventing movement of the barrel in a distal-proximal direction and enabling rotation of the barrel about a longitudinal axis of the barrel;
a contact sensor positioned on the base, the contact sensor comprising a sensor element positioned adjacent the flange of the barrel when the barrel is retained by the barrel holder, with the contact sensor configured to measure a distance between the sensor element and an underside of flange, so as to obtain a measurement value; and
a digital comparator operatively connected to the contact sensor to receive measurement values therefrom;
wherein the contact sensor obtains a plurality of measurement values as the barrel is rotated on the barrel holder; and
wherein the digital comparator:
receives the plurality of measurement values from the sensor;
determines maximal - minimal measurement values from the plurality of measurement values; and
calculates an under-flange flatness measurement for the flange from the maximal - minimal measurement values.

2. The tool of claim 1, wherein the base comprises:
a base plate; and
an end plate positioned at one end of the base plate and extending upwardly therefrom.

3. The tool of claim 2, wherein the barrel holder comprises:
a plurality of bearings provided on the base plate and arranged to engage the cylindrical main body;
a wheel suspended above the base plate and positioned above the plurality of bearings, such that the barrel is positionable between the plurality of bearings and the wheel, the wheel configured to engage the cylindrical main body to enable rotation thereof on the plurality of bearings responsive to rotation of the wheel; and
a stop flange provided at one end of the plurality of bearings, opposite from the end plate, the stop flange configured to engage a top side of the flange when the barrel is positioned on the plurality of bearings;
wherein, when loaded on the barrel holder, the barrel is engaged by the stop flange and the end plate at opposing ends thereof, to prevent movement of the barrel in the distal-proximal direction.

4. The tool of claim 3, wherein the wheel is mounted on a support arm, with the support arm supported and retained by an arm holder affixed to the base plate, and wherein the wheel is rotatably mounted on the support arm via a wheel bearing.

5. The tool of claim 3, wherein the wheel comprises an outer rubber surface configured to grip the cylindrical main body, to facilitate rotation of the barrel when the wheel is rotated.

6. The tool of claim 3, wherein the stop flange is positioned at a distal end of a spring-loaded arm configured to move the stop flange in the distal-proximal direction to selectively bring the stop flange into and out of contact with the flange of the barrel.

7. The tool of claim 6, wherein the spring-loaded arm is mounted retained on a mounting block that is secured on the base plate, with the spring-loaded arm extending through an opening of the mounting block and movable relative to the mounting block in the distal-proximal direction.

8. The tool of claim 7, wherein the spring-loaded arm comprises:
a handle positioned at a proximal end of a spring-loaded arm, the handle configured to be engaged by a user; and
a spring is positioned about the spring-loaded arm at a location between the mounting block and the stop flange, with the spring biasing the arm to a distal location where the stop flange is brought into contact with the flange of the barrel.

9. The tool of claim 3, wherein the plurality of bearings comprises a distal pair of bearings and a proximal pair of bearings.

10. The tool of claim 2, wherein the contact sensor comprises a sensor key on which the sensor element is mounted, with the sensor key mounted on rods secured to the end plate.

11. The tool of claim 10, wherein the sensor key is repositionable along a length of the rods in order to properly position the sensor element relative to the underside of the flange.

12. The tool of claim 1, wherein the digital comparator is configured generate an output indicating that the barrel is defective if the under-flange flatness measurement falls outside of an acceptable tolerance range.

13. The tool of claims 12, wherein the acceptable tolerance range of the under-flange flatness measurement is +/- 0.4 mm.

14. A method for measuring under-flange flatness of a medical injection device barrel using the tool of claim 1, the method comprising:
loading the barrel into the barrel holder of the tool;
activating the contact sensor to ready the contact sensor for acquisition of measurements on the under-flange flatness of the barrel;
rotating the barrel in the barrel holder a full 360° about a longitudinal axis of the barrel;
as the barrel is being rotated about the longitudinal axis, continuously acquiring measurement values of the under-flange flatness of the barrel via the contact sensor;
providing the measurement values to the digital comparator, with the digital comparator:
analyzing the measurement values via the digital comparator to determine maximal - minimal values; and
generating an under-flange flatness measurement for the flange from the maximal - minimal values.

15. The method of claim 14, wherein the barrel holder comprises a plurality of bearings, a support arm including a wheel thereon, and a spring-loaded arm comprising a stop flange and a handle, and wherein loading the barrel into the barrel holder comprises:
actuating the support arm to change the positioning of the wheel and provide access to the plurality of bearings;
actuating the spring-loaded arm by pulling the handle, to move the stop flange proximally and provide access to the plurality of bearings;
positioning the barrel onto the plurality of bearings and with a tip of the barrel in contact with an end plate of the base; and
upon such placement of the barrel onto the plurality of bearings:
releasing the spring-loaded arm to bring the stop flange into contact with the flange of the barrel; and
actuating the support arm to bring the wheel into contact with the cylindrical main body of the barrel.
